# EUROPÄISCHE PATENTSCHRIFT

(11) **EP 1 096 892 B1**
(45) Veröffentlichungstag und Bekanntmachung des Hinweises auf die Patenterteilung: **10.11.2004**
(21) Anmeldenummer: 99934650.5
(22) Anmeldetag: 09.07.1999
(51) Int. Cl.: A61B 17/86, F16B 39/28

(54) **KNOCHENSCHRAUBE, INSBESONDERE FÜR DEN EINSATZ BEI TRANSLAMINÄRER WIRBELVERSCHRAUBUNG**
OSTEOSYNTHESIS SCREW, ESPECIALLY FOR APPLICATION BY A TRANSLAMINAR VERTEBRAL SCREW
VIS D'OSTEOSYNTHESE, S'UTILISANT NOTAMMENT LORS D'UNE FIXATION PAR VIS VERTEBRALE TRANSLAMINAIRE

(30) Priorität: 13.07.1998 DE 19831336
(43) Veröffentlichungstag der Anmeldung: 09.05.2001
(73) Patentinhaber: Sepitec Foundation, 9490 Vaduz (LI)
(72) Erfinder: MAGERL, Fritz, CH-9011 St. Gallen (CH); WINTERMANTEL, Erich, CH-5442 Fislisbach (CH); MAYER, Jörg, CH-5702 Niederlenz (CH); TOGNINI, Roger Roland, CH-9443 Widnau (DE); DRANSFELD, Clemens, CH-5600 Lenzburg (CH); SPIRIG, Walter, CH-9428 Platz-Walzenhausen (CH)
(74) Vertreter: Menges, Rolf, Dipl.-Ing.
(86) Internationale Anmeldenummer: PCT/EP1999/004846
(87) Internationale Veröffentlichungsnummer: WO 2000/003649

(56) Entgegenhaltungen:
- EP-A- 0 424 734
- US-A- 4 820 098
- US-A- 5 672 178
- M.H.HEGGENESS UND S.I.ESSES: "Translaminar Facet Joint Screw Fixation for Lumbar and Lumbosacral Fusion" SPINE, Bd. 16, Nr. 6, 1. Juni 1991 (1991-06-01), Seiten S266-S269, XP000617673

## Beschreibung

Die Erfindung betrifft eine Knochenschraube, insbesondere für den Einsatz bei translaminärer Wirbelverschraubung, mit einem Schaft sowie einem an wenigstens einem Ende ausgebildeten Kopf mit einem Werkzeugangriff, wobei der Schaft an dem dem Kopf zugewandten Endabschnitt einen mit einem Gewinde versehenen Bereich aufweist.

Translaminäre Verschraubungen werden an der Lendenwirbelsäule im Rahmen von Versteifungsoperationen (Spondylodesen) und der operativen Behandlung von Verletzungen seit fast zwanzig Jahren angewendet. Das Prinzip der translaminären Verschraubung besteht darin, die zwischen den Wirbeln möglichen Bewegungen durch Blockierung der Zwischenwirbelgelenke mit Hilfe von Knochenschrauben zu unterbinden, um mit der dadurch bewirkten Ruhigstellung des betreffenden Abschnitts der Lendenwirbelsäule die Konsolidierung der Spondylodese bzw. Heilung der Verletzung zu sichern. Die Schrauben treten auf der einen Seite des Dornfortsatzes des Knochens ein, ziehen durch die gegenseitige Lamina, durchqueren das intervertebralgelenk und enden in der Basis des Querfortsatzes des unteren Wirbels. Das freie Ende der Schraube liegt dann in der ventrokaudalen Begrenzung der Querfortsatzbasis, darf dort aber nicht vorstehen.

Zur Plazierung translaminärer Schrauben in den dorsalen Strukturen der Wirbel steht nur wenig Platz zur Verfügung. Aus anatomischen Gründen gibt es sehr wenig Toleranzen hinsichtlich der sowohl in anatomischer als auch in mechanischer Hinsicht günstigsten Schraubenlage. Schon geringe Abweichungen aus der Ideallage sind mit einer rasch abnehmenden Wirksamkeit der betreffenden Schraube verbunden. Aus diesem Grund wird für die Plazierung der Schrauben die Verwendung eines speziellen Zielgerätes empfohlen.

Aus der Tatsache, dass es in einem Wirbel jeweils nur eine ldeallage für jede der beiden translaminären Schrauben gibt, resultiert im Bereich des Domfortsatzes des oberen Wirbels, d.h. dort, wo die Schrauben in den Knochen eingesetzt werden, wo sie sich kreuzen und wo sie nur nebeneinander liegen können, das Problem, dass nicht beide Schrauben ideal positioniert werden können, so dass schon beim Setzen der ersten Schraube die Lage der zweiten Schraube zu berücksichtigen ist. Andernfalls könnte für die zweite Schraube nur mehr eine sehr ungünstige Platzierungsmöglichkeit verbleiben. Wenig Spielraum steht vor allem am fünften Lendenwirbel zur Verfügung.

Die Schrauben wirken im Prinzip wie Stifte, welche die Wirbelgelenke blockieren. Da glatte Stifte im Knochen rutschen würden und anderes nicht zur Verfügung stand, wurden nach dem allgemeinen Fachwissen des Erfinders Dr. Magerl bisher normale Knochenschrauben mit durchgehendem Gewinde verwendet. Dies hat aber einige Nachteile: Bei härteren Knochen muss das Gewinde vorgeschnitten werden, was umständlich ist und die Gefahr einer Nervenverletzung erhöht. Wegen des hinsichtlich der mechanischen Festigkeitswerte nutzlosen Gewindes ist der Außendurchmesser der Schraube unnötig groß. Da im Bereich der Zwischenwirbelgelenke während des mehrere Monate beanspruchenden Heilungsprozesses quer zur Schraubenachse gerichtete Kräfte in großer Zahl auf die Schrauben und deren Lager einwirken, können diese Lager durch die scharfen Gewindekanten mit der Zeit zerstört werden. Schrauben verleiten auch zum festen Andrehen. Weil das Gewinde translaminärer Schrauben durchgehend vorgeschnitten ist, also auch in den Dornfortsatz und die daran anschließende Lamina, kann bei festem Andrehen der Schraube der Domfortsatz abbrechen. Ein gravierender Nachteil der herkömmlichen translaminären Verschraubung besteht darin, dass, weil die Schrauben in den Domfortsätzen nebeneinander liegen müssen und der Platz hier knapp ist, bei der Plazierung der ersten Schraube schon die der zweiten Schraube berücksichtigt werden muss. Andernfalls kann es passieren, dass nur eine Schraube in anatomischer und mechanischer Hinsicht günstig liegt. Schließlich wird der Domfortsatz durch zwei nebeneinander liegende Schraubenkanäle geschwächt. Letzteres wirkt sich ungünstig aus, wenn ein zusätzliches lastaufnehmendes Element, z.B. ein Knochenspan, zwischen den Domfortsätzen eingeklemmt werden soll. Der vorstehende Kopf konventioneller, bisheriger Schrauben macht eine versenkte Anordnung unmöglich. Die Schrauben sollen später wieder entfernt werden können.

Bei einer bekannten Schraube zur Befestigung von Prothesen an Knochen (EP-A-0424734) sind ein Kopf und ein gewindetragender Schaft vorgesehen, wobei das Gewinde zwei verschiedene Typen aufweist. Das zweite Gewinde ist selbstschneidend ausgeführt und vor allem weist der Schaft der Schraube einen insgesamt kegelstumpfförmigen Kern auf. Der Kopf ist zylindrisch ausgeführt. Der erste Teil des Gewindes wird zur Befestigung im trabecularen Gewebe eines Knochens herangezogen, wogegen mit dem zweiten Gewinde der corticale Teile des Knochens gegenüber dem Teil fixiert werden soll, in den die Schraube eingeführt ist. Es ist hier wohl eine Schraube der eingangs geschilderten Art vorhanden, jedoch ist hier praktisch über die ganze Länge des Schaftes mit Ausnahme des Schraubenkopfes ein Gewinde vorhanden, wobei durch die mehrfach voneinander abgesetzten Gewindebereiche grundsätzlich andere Wirkungen erzielt werden als dies bei einer Schraube bei der tanslaminären Wirbelverschraubung erforderlich ist.

Die vorliegende Erfindung hat sich zur Aufgabe gestellt, eine Knochenschraube der eingangs genannten Art so auszubilden, dass sie knochenschonend eingedreht werden kann, wobei außerdem eine Sicherung in axialer Richtung und in Drehrichtung gewährleistet sein soll und eine Verbesserung der Anwendungstechnik erreicht werden kann.

Erfindungsgemäß wird dazu vorgeschlagen, dass der auf den mit dem Gewinde versehenen Bereich folgende restliche Bereich des Schaftes bis zum freien Ende gewindefrei ausgeführt ist, dass der Kopf einen zumindest annähernd dem Außendurchmesser des Gewindes entsprechenden Durchmesser aufweist und mit einem Innenwerkzeugangriff versehen ist, und dass der Bereich des Schaftes mit dem Gewinde und/oder der gewindelose Bereich des Schaftes unrund, nach Art eines Gleichdicks, z.B. trilobular, oder mehreckig ausgestaltet und/oder mit Erhebungen und/oder Vertiefungen versehen ist.

Durch die Ausbildung eines Gewindes nur in einem Teilbereich des Schaftes und durch einen großen gewindelosen Bereich kann bei größerem Kemdurchmesser die Schädigung der Knochenwandung sehr gering gehalten werden. Das Gewinde ist also nur vorgesehen, um eben ein Verschieben der Knochenschraube in axialer Richtung zu verhindern. Wenn dann noch hinzukommt, dass der Bereich mit dem Gewinde und/oder der gewindelose Bereich unrund od.dgl. ausgeführt sind, wird unmittelbar nach dem Setzen der Schraube durch elastische Anpassung des elastisch deformierbaren Knochens von Anfang an und später durch nachwachsenden Knochen ein Formschluss zwischen dem Knochenmaterial und der Oberfläche der Knochenschraube erzielt. Die neue Schraube ist daher schon von Anfang an und dann zunehmend gegen das sonst bei herkömmlichen Knochenschrauben infolge repetitiver Lastwechsel vorkommende selbsttätige Ausdrehen gesichert. Weiter ist durch die besondere Ausgestaltung des Kopfes die erfindungsgemäße Knochenschraube versenkbar, kann also zur Gänze in die gegenseitig zu blockierenden Knochenteile eingreifen, steht also nicht störend vor. Es kann damit auch nicht zu gefährlichen Bruchsituationen kommen, wenn beim Eindrehen der Kopf auf einem Knochenteil aufliegen würde.

Da für eine Verdrehsicherung der Knochenschraube keine großen Kräfte notwendig sind, reicht der Formschluß durch elastische Verformung und nachwachsendes Knochengewebe aus. Damit ist aber auch die Möglichkeit des nachträglichen Entfernens der Knochenschraube gegeben, da eine Krafteinwirkung in Drehrichtung der Knochenschraube problemlos diesen Formschluß aufhebt.

Durch die erfindungsgemäßen Maßnahmen sind zwei Anwendungstechniken möglich geworden. Wo genügend Platz vorhanden ist, können die Schrauben auf herkömmliche Art implantiert werden, d.h. mit an der Oberfläche des Domfortsatzes freiliegendem Kopfstück und sich im Domfortsatz kreuzend. Als zweite und in vielen Fällen günstigere Technik hat man die Möglichkeit, zuerst eine kürzere Schraube einzusetzen, deren Kopfteil versenkt ist. Die zweite Schraube kann dann ohne Rücksicht auf die Lage der ersten Schraube implantiert werden. Der Vorteil dieser Technik besteht darin, daß hinsichtlich der günstigsten anatomischen und mechanischen Schraubenlage keine Kompromisse nötig sind. Beide Schrauben können optimal plaziert werden.

Gerade bei einer solchen Knochenschraube ist es vorteilhaft, wenn das Gewinde auf dem Schaft selbstfurchend und/oder selbstschneidend ausgebildet ist.

Ein besonderes Ausführungsbeispiel sieht vor, daß der gewindefreie Bereich des Schaftes zylindrisch oder konisch ausgeführt ist, wobei der Bereich des Schaftes mit dem Gewinde unrund, nach Art eines Gleichdicks, z.B. trilobular, oder mehreckig ausgestaltet und/oder mit Erhebungen und/oder Vertiefungen versehen ist. Es reicht für eine Verdrehsicherung völlig aus, wenn nur der kurze Bereich des Gewindes z.B. trilobular ausgeführt ist. Außerdem kann dann auch noch das Eindrehen der Knochenschraube und somit die Herstellung des Gewindes im Bohrloch im Knochen erleichtert werden. Eine solche Ausführung ist insofern auch noch vorteilhaft, als es in dem tief eingedrehten Bereich der Knochenschraube nur zur Anlage des Knochenmaterials an einem zylindrischen Bereich kommen kann, also hier kein die Verdrehung behindernder Formschluß stattfinden kann. Dies ist für ein nachträgliches Lösen und Herausdrehen der Knochenschraube vorteilhaft.

Im Rahmen der Erfindung ist es aber auch möglich, daß das Gewinde und der Schaft in diesem Bereich zylindrisch ausgeführt sind, wobei der an den Bereich mit dem Gewinde anschließende gewindefreie Bereich des Schaftes unrund, nach Art eines Gleichdicks, z.B. trilobular, oder mehreckig ausgestaltet und/oder mit Erhebungen und/oder Vertiefungen versehen ist. Eine solche Ausführung wäre z.B. dann denkbar, wenn das tiefer liegende Knochenmaterial oder Knochengewebe auch bezüglich des nachwachsenden Materials nicht zu starr ist. Für eine Verdrehsicherheit ist auch diese Variante vorteilhaft, wobei jedoch auch immer die notwendige Möglichkeit des Herausdrehens der Knochenschraube offen bleiben muß. Für die axiale Sicherung ist auch hier wieder der Bereich mit dem Gewinde vorgesehen.

Weiters wird vorgeschlagen, daß der Bereich des Schaftes mit dem Gewinde unmittelbar an den Kopf anschließend angeordnet ist. Für einen knochenschonenden Einsatz ist es besonders vorteilhaft, wenn eben das Gewinde nicht zu tief in den Knochen hinein hergestellt werden muß. Für einen großen Abschnitt beim Setzen der Knochenschraube genügt also lediglich eine Art Einschieben.

Damit auf die gesamte Länge des hergestellten Bohrloches eine echte Blockierung der miteinander zu verbindenden Knochenteile erfolgen kann, ist vorgesehen, daß der Außendurchmesser des gewindefreien Bereiches des Schaftes zumindest annähernd dem Kerndurchmesser des Bereiches mit dem Gewinde entspricht. Es ist also über die ganze Länge der Knochenschraube eine gleichmäßig Abstützung an der Bohrungswandung gegeben, wobei das Gewinde nur zusätzlich in die Bohrungswandung eingreift als Verschiebesicherung in axialer Richtung.

Da auch schon ein kurzer Bereich des Eingriffes eines Gewindes ausreicht, um die axiale Sicherung der Knochenschraube zu bewirken, wird vorgeschlagen, daß die axiale Länge des Bereiches mit dem Gewinde kleiner ist als die axiale Länge des gewindefreien Bereiches des Schaftes.

Da infolge des versenkbaren Kopfes der Knochenschraube nur ein Innenwerkzeugangriff in Frage kommt, muß gerade für die Notwendigkeit des nachträglichen Herausdrehens der Knochenschrauben Sorge getragen werden. Es kann ja immer wieder vorkommen, daß allein schon beim Einführen des Werkzeuges Knochenmaterial od.dgl. in den Innenwerkzeugangriff eindringt. Es wird daher vorgeschlagen, daß der Innenwerkzeugangriff am Kopf an dessen tiefster Begrenzung eine zusätzliche Ausnehmung oder eine taschenartige Vertiefung aufweist. Es wird dadurch ein kleiner Aufnahmeraum geschaffen, um solches unbeabsichtigt in den Innenwerkzeugangriff gelangtes Material aufnehmen zu können, damit auch der Eingriff des Werkzeuges nicht gestört wird.

Eine weitere Ausführungsmöglichkeit sieht vor, daß der Kopf an seinem freien Ende einen ringförmigen Bund mit gegenüber dem Außendurchmesser des Kopfes verringertem Durchmesser aufweist. Durch diese vorteilhafte Konstruktion ergeben sich gerade im Hinblick auf eine bessere Handhabbarkeit solcher Knochenschrauben neue Möglichkeiten im Zusammenwirken mit entsprechenden Werkzeugen, aber auch im Zusammenwirken mit eventuell vorzusehenden Schutzelementen oder Abdeckkappen.

Um dabei aber keine Querschnittsschwächung hervorzurufen, wird vorgeschlagen, daß die axiale Länge des ringförmigen Abschnittes weniger als die halbe Gesamtlänge, vorzugsweise weniger als ein Drittel der Gesamtlänge des Kopfes beträgt.

Eine vorteilhafte Ausbildung sieht vor, daß der ringförmige Abschnitt an eine auf ein Werkzeug aufgesetzte Ringmanschette angepaßt ist, welche beim Schraubvorgang den ringförmigen Abschnitt des Kopfes umfaßt. Es ist dadurch eine optimale Halterung der einzudrehenden Knochenschraube an dem Werkzeug möglich, es wird also ein genaues Zielen mit der Knochenschraube erreicht. Durch eine solche Ringmanschette kann die Knochenschraube auch unverlierbar am Werkzeug gehalten werden, bis diese in das Bohrloch im Knochen eingeführt ist. Außerdem wird dadurch der ringförmige Bund bis zum Herausziehen des Werkzeuges und somit auch der Manschette von Verunreinigungen und Knochenablagerungen frei gehalten.

Damit ein leichteres Einführen der Knochenschraube in das vorbereitete Bohrloch möglich ist, wird vorgeschlagen, daß am freien Ende des gewindefreien Bereiches des Schaftes ein z.B. kegelstumpf- oder halbkugelförmig sich verjüngender Abschnitt ausgebildet ist.

Eine zusätzliche Ausführungsvariante sieht eine kraft- und/oder formschlüssig auf den Kopf aufsetzbare Abdeckkappe zum Verschließen des Innenwerkzeugangriffes vor. Damit kann der freie Raum des Innenwerkzeugangriffes für ein nachträglich wieder notwendiges Herausdrehen der Knochenschraube frei gehalten werden. Das nachwachsende Knochenmaterial kann daher auch nicht in diesen Bereich des Innenwerkzeugangriffes vordringen.

In diesem Zusammenhang ist es vorteilhaft, wenn die Abdeckkappe einen in Richtung zum Kopf weisenden ringförmig verlaufenden Steg aufweist, welcher auf den ringförmigen Bund verringerten Durchmessers am Kopf aufschiebbar ist. Damit ist eine optimale Halterung der Abdeckkappe gewährleistet und außerdem wird auch der ringförmige Bund von nachwachsendem Knochenmaterial frei gehalten. Nach dem Entfernen einer solchen Abdeckkappe kann unmittelbar wieder das Werkzeug zusammen mit der Ringmanschette angesetzt werden.

Es ist dabei vorteilhaft, wenn der Außendurchmesser der Abdeckkappe zumindest annähernd gleich ist dem Außendurchmesser des Kopfes. Dies erleichtert das Aufbringen der Abdeckkappe nach dem Setzen der Knochenschraube, da praktisch ein auf des Bohrloch angepaßter Teil eingesetzt wird. Natürlich wird dadurch auch das spätere Entfernen der Abdeckkappe erleichtert, Wenn gemäß einer weiteren Ausführungsvariante die Abdeckkappe in Achsrichtung gesehen nach beiden Seiten hin abstehende ringförmig verlaufende Stege aufweist, dann ist eine Möglichkeit geschaffen, eine solche Abdeckkappe auch mit einem Werkzeug auf das freie Ende des Kopfes der Knochenschraube aufstecken zu können. Mit dem auf die andere Seite abstehenden ringförmigen Steg kann das Ende eine Werkzeuges umschlossen werden, mit welchem dann die Abdeckkappe auf den bereits versenkt im Bohrloch befindlichen Kopf aufgedrückt werden kann.

Im Zusammenhang mit der auf den Kopf der Knochenschraube nach deren Eindrehen aufgebrachten Abdeckkappe wird auch eine optimale Lösung für das spätere Abziehen bzw. Entfernen der Abdeckkappe vorgeschlagen. Diese Lösung sieht vor, daß die Abdeckkappe in ihrem Zentrum eine Solldurchbruchstelle und/oder z.B. sektorförmig über Sollbruchlinien verbundene Abschnitte zur Herstellung einer Öffnung beim Einsatz eines Werkzeuges mit einer kegel- oder pyramidenförmigen Spitze aufweist. Es kann also in einfacher Weise ein Werkzeug eingeschoben werden, mit welchem die Abdeckkappe durchdrungen wird. Nach Art eines Widerhakens kann dann dieses Werkzeug die Abdeckkappe in deren Zentumsbereich hintergreifen, wobei durch Zurückziehen des Werkzeuges die Abdeckkappe vom Kopf abgezogen werden kann. Es ist dann nur noch das Werkzeug für den innenwerkzeugangriff in der Knochenschraube einzusetzen, worauf dann die Knochenschraube wieder herausgedreht werden kann. Dabei ist nach dem Abziehen der Abdeckkappe nicht nur der Innenwerkzeugangriff, sondern auch der ringförmige Bund ohne Ablagerungen frei zugänglich.

Wenn die Abdeckkappe an ihrer Außenbegrenzung einen oder mehrere rillenartige Vertiefungen aufweist, dann ist auch eine unverlierbare Halterung derselben in der auf dem Kopf der Knochenschraube aufgesetzten Stellung gewährleistet. Das nachwachsende Knochenmaterial wird sofort auch formschlüssig in diese rillenartige(n) Vertiefung(en) eingreifen und eine nachträgliche axiale Verschiebung gegenüber dem Knochen und damit natürlich auch gegenüber der Knochenschraube verhindern.

Eine weitere Ausführungsvariante ist gekennzeichnet durch einen den nach dem versenkten Setzen der Knochenschraube freibleibenden Abschnitt der Bohrung zumindest teilweise ausfüllenden Zapfen aus einem elastischen, gewebeverträglichen, nichtresorbierbaren Material. Auf diese Weise kann immer eine Zugänglichkeit zum Schraubenkopf gewährleistet werden, selbst wenn nach langer Zeit die Knochenschraube wieder entfernt werden soll. Es kann damit auch verhindert werden, daß der freibleibende Bereich der Bohrung durch das eindringende Knochengewebe zuwächst und somit praktisch verschlossen wird.

Eine weitere Ausgestaltung ist gekennzeichnet durch ein in den Innenwerkzeugangriff der Knochenschraube einsetzbares Werkzeug, auf welches koaxial eine Hülse aufgesetzt ist, welche wenigstens einen Großteil der axialen Länge des Kopfes der Knochenschraube koaxial umfassend aufnimmt. Durch diese Ausgestaltung kann eine optimale Ausrichtung und Halterung der Knochenschraube beim Eindrehen bewirkt werden. Ein Verkanten zwischen Schraube und Werkzeug ist somit beim Eindrehen auszuschließen.

Weitere Merkmale und Vorteile werden anhand eines in den Zeichnungen dargestellten Ausführungsbeispieles in der nachstehenden Beschreibung noch näher erläutert. Es zeigen:
Fig.1 eine Ansicht einer Knochenschraube in vergrößerter Darstellung;
Fig.2 ein Wirbel mit Wirbelgelenken in Draufsicht, wobei in etwa die Lage der eingesetzten Knochenschrauben ersichtlich ist;
Fig.3 eine gegenüber Fig. 2 vergrößerte Darstellung eines Teilbereiches des Wirbelbogens in Draufsicht, wobei in etwa die Lage der eingesetzten Knochenschrauben ersichtlich ist, und wobei in einem freibleibenden Bereich der Bohrung ein Zapfen eingesetzt ist;
Fig.4 eine gegenüber Fig.1 vergrößerte Darstellung des mit einem Gewinde versehenen Bereiches des Schaftes der Knochenschraube;
Fig.5 einen Schnitt nach der Linie V-V- in Fig.4;
Fig.6 eine Draufsicht auf den Kopf der Knochenschraube;
Fig.7 eine Seitenansicht eines Werkzeuges zum Drehen der Knochenschraube;
Fig.8 in vergrößerter Darstellung den Bereich des Kopfes der Knochenschraube;
Fig.9 eine Darstellung mit einem in den Kopf der Knochenschraube eingesetzten Werkzeug;
Fig.10 eine auf den Kopf aufsetzbare Abdeckkappe in einem Längsschnitt dargestellt;
Fig.11 einen Schnitt durch den Bereiche des Kopfes mit aufgesetzter Abdeckkappe;
Fig.12 ein Werkzeug zur Handhabung beim Aufsetzen der Abdeckkappe mit einer am freien Ende des Werkzeuges aufgesetzter Abdeckkappe;
Fig.13 ein Werkzeug zum Herausziehen der Abdeckkappe;
Fig.14 ein Werkzeug gemäß Fig.13 im Einsatz nach dem Durchdrücken der Abdeckkappe und vor dem Abziehen der Abdeckkappe;
Fig.15 eine weitere Ausführungsvariante eines Werkzeuges zum Eindrehen der Knochenschraube;
Fig.16 eine andere Ausführungsform einer auf den Kopf einer Schraube aufsetzbaren Kappe;
Fig.17 einen Zapfen in Ansicht, wie er beim Beispiel nach Fig.3 zum Einsatz kommt.

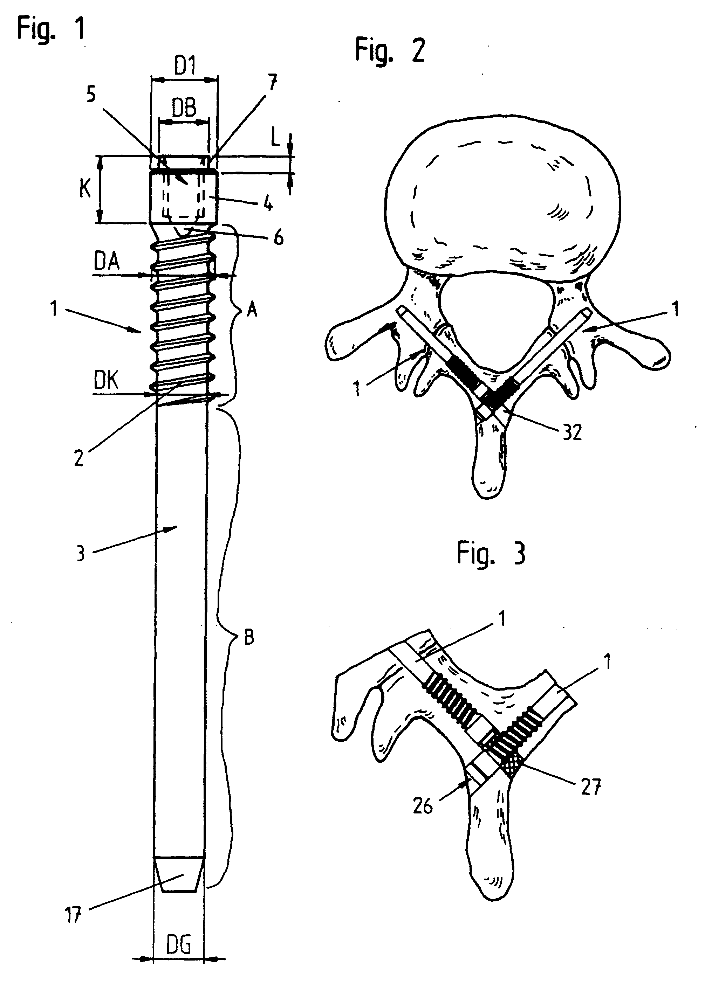

Die in Fig.1 dargestellte Knochenschraube 1 ist insbesondere für den Einsatz bei translaminärer Wirbelverschraubung vorgesehen. Diese Knochenschraube 1 besteht aus einem zumindest teilweise mit einem Gewinde 2 versehenen Schaft 3 sowie einem an wenigstens einem Ende ausgebildeten Kopf 4 mit einem Werkzeugangriff 5. Der Schaft 3 weist an dem dem Kopf 4 zugewandten Endabschnitt einen mit einem Gewinde 2 versehenen Bereich A auf. Der restliche Bereich B des Schaftes 3 bis zum freien Ende ist gewindefrei ausgeführt. Der Kopf 4 weist einen zumindest annähernd dem Außendurchmesser DA des Gewindes 2 entsprechenden Durchmesser DK auf. Ferner ist der Kopf 4 mit einem Innenwerkzeugangriff 5 versehen.

Der gewindefreie Bereich B des Schaftes 3 ist zylindrisch ausgeführt und der Bereich A des Schaftes 3 mit dem Gewinde 2 ist nach Art eines Gleichdicks, im besonderen Fall hier mit einem trilobularen Querschnitt versehen.

Es ist aber grundsätzlich möglich, daß der Bereich A des Schaftes 3 mit dem Gewinde 2 und/oder der gewindelose Bereich B des Schaftes 3 unrund, nach Art eines Gleichdicks, z.B. trilobular, oder mehreckig ausgestaltet und/oder mit Erhebungen und/oder Vertiefungen versehen ist. So könnte also auch das Gewinde 2 und der Schaft 3 in diesem Bereich A zylindrisch ausgeführt sein, wobei dann der an den Bereich A mit dem Gewinde 2 anschließende gewindefreie Bereich B des Schaftes 3 über seine ganze Länge oder aber nur über einen oder mehrere Teilabschnitte unrund, nach Art eines Gleichdicks, z.B. trilobular, oder mehreckig ausgestaltet und/oder mit Erhebungen und/oder Vertiefungen versehen ist. Das Gewinde 2 auf dem Schaft 3 ist vorteilhaft selbstschneidend und/oder selbstfurchend ausgebildet.

Der Bereich A des Schaftes 3 mit dem Gewinde 2 ist unmittelbar an den Kopf 4 anschließend angeordnet. Ein besonderes Merkmal dieser Knochenschraube 1 ist die zumindest annähernde Anpassung des Außendurchmessers DG des gewindefreien Bereiches B des Schaftes 3 an den Kerndurchmesser DK des Bereiches A mit dem Gewinde 2. Die axiale Länge des Bereiches A mit dem Gewinde 2 ist beim gezeigten Ausführungsbeispiel kleiner als die axiale Länge des gewindefreien Bereiches B des Schaftes 3.

Es wurde schon erwähnt, daß der Kopf 4 der Knochenschraube 1 mit einem Innenwerkzeugangriff 5 versehen ist. Zur Aufnahme von zufällig in diesen Innenwerkzeugangriff hineingefallenen Knochenresten od.dgl. weist der Innenwerkzeugangriff 5 an dessen tiefster Begrenzung eine zusätzliche Ausnehmung oder eine taschenartige Vertiefung 6 auf. Der Kopf 4 weist an seinem freien Ende einen ringförmigen Bund 7 mit gegenüber dem Außendurchmesser D1 des Kopfes 4 verringertem Durchmesser DB auf. Die axiale Länge L des ringförmigen Bundes 7 beträgt weniger als die halbe Gesamtlänge K, vorzugsweise weniger als ein Drittel der Gesamtlänge K des Kopfes 4. Der ringförmige Bund 7 kann zur noch besseren Haftung einer Abdeckkappe 18 mit einer Riffelung oder einer Aufrauhung versehen sein. Das Wesen dieses ringförmigen Bundes 7 wird noch näher erläutert. Am freien Ende des gewindefreien Bereiches B des Schaftes 3 ist ein kegelstumpfförmig sich verjüngender Abschnitt 17 ausgebildet. Dadurch ist die Knochenschraube 1 leichter in das vorbereitete Loch einzuführen.

Die Knochenschraube 1 kann aus Metall, z.B. aus rostfreien Stahl, Titan, Tantal, CoCr-Legierung oder aus einem anderen Metall, gefertigt sein. Es ist aber gerade auch eine Fertigung aus einem Verbundwerkstoff vorteilhaft, der aus Polymeren und entsprechenden Verstärkungsfasern oder aber aus Keramikwerkstoffen gebildet ist. Bei einer Fertigung aus einem Verbundwerkstoff werden in sinnvoller Weise auch Fasern und/oder faserartige Teile aus einem Material mit hoher Röntgenabsorbtion eingesetzt, also z.B. aus Tantal, Wolfram, Gold, Platin od.dgl. Durch entsprechende Ausrichtung und Menge dieser Fasern ist auch die Röntgensichtbarkeit der eingesetzten Knochenschrauben einstellbar. Die beschriebene Knochenschraube ist auch für andere Bereiche der Medizintechnik einsetzbar, wo es in besonderer Weise darauf ankommt, daß nicht ein allzu großer Bereich auf die Länge des eingesetzten Befestigers durch eine Gewindeeinformung beeinträchtigt wird und wo es auch darauf ankommt, daß eine Verdrehsicherung und ein axiale Sicherung gewährleistet ist und daß der Kopf versenkt angeordnet werden kann.

In der Regel wird vorerst mittels eines Bohrers in den entsprechenden Knochenbereichen ein Loch für die anschließende Aufnahme einer Knochenschraube 1 hergestellt. Bei translaminärer Verschraubung ist das Vorbohren ohne ein Zielgerät wohl möglich, aber nicht zu empfehlen. Nach dem Eindrehen der Knochenschrauben 1 in die kleinen Wirbelgelenke sind diese zur Gänze versenkt angeordnet. Durch den trilobularen Querschnitt des Gewindes 2 wird infolge des rasch nachwachsenden Knochengewebes eine Verdrehsicherung geschaffen. Das Gewinde 2 selbst bewirkt eine Sicherung gegen ein Verschieben in axialer Richtung.

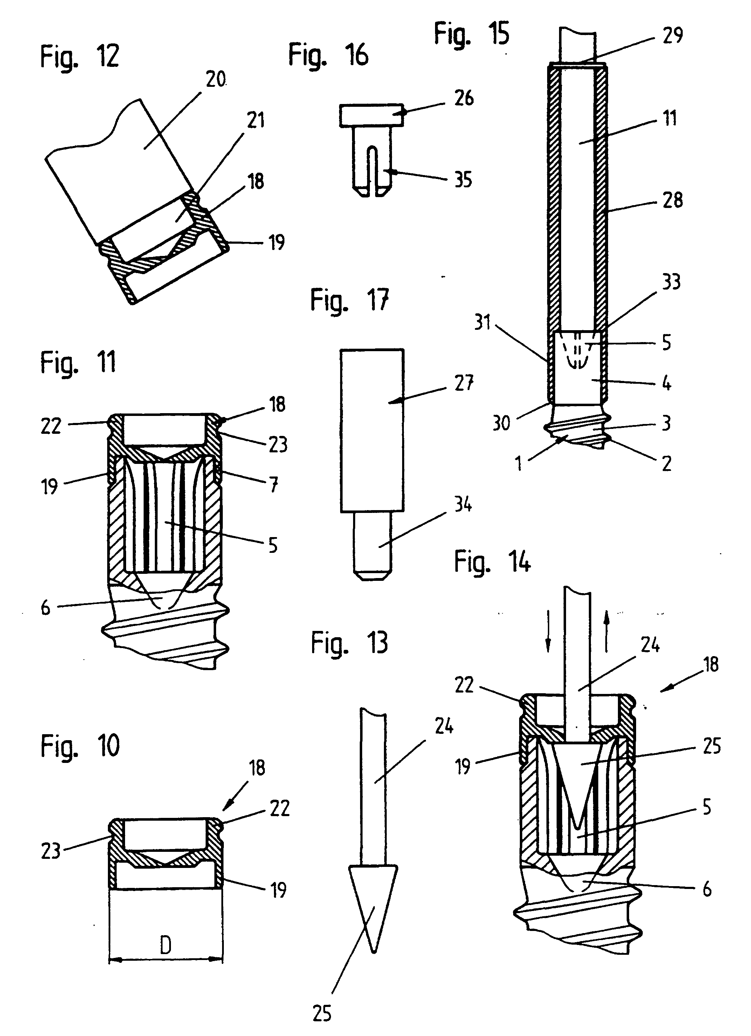

Wie aus der Fig. 3 ersichtlich ist, sind bei translaminärer Verschraubung auch Einsätze erforderlich, bei welchen ein verschieden tiefes Versenken der ersten Knochenschrauben 1 notwendig ist, um auch die zweite Knochenschraube 1 ohne Behinderung durch die erste Schraube an günstigster Stelle plazieren zu können. Es wird dann vorerst eine Bohrung für die erste, tiefer versenkt anzubringende Schraube hergestellt und diese Schraube eingedreht. Dann wird die zweite Bohrung hergestellt. Nun wird aber nicht unmittelbar diese zweite Schraube eingesetzt, sondern es wird zuerst der hinter dem Kopf der Schraube freibleibende Bereich der ersten Bohrung 32 mittels eines Zapfens 27, wie er auch aus Fig.17 ersichtlich ist, gefüllt. Der Zapfen kann im Wesentlichen als zylindrischer Teil ausgeführt sein, welcher an seinem einen freien Ende einen Fortsatz 34 aufweist, welcher in den Innenwerkzeugangriff 5 des Kopfes 4 der Knochenschraube eingreifen kann, um diesen vor dem Eindringen von Gewebematerial zu schützen. Dieser Zapfen 27 besteht aus einem elastischen, gewebeverträglichen, nichtresorbierbaren Material, z.B. Silikon-Kautschuk. Es ist aber auch der Einsatz eines elastisch/plastischen Materials möglich. Nach dem Einsetzen dieses Zapfens 27, der infolge des elastischen Materials auch optimal in die Öffnung des Innenwerkzeugangriffes 5 der Schraube eingreift, wird der die zweite Bohrung kreuzende Bereich des Zapfens 27 beispielsweise mittels einer Ahle durchstoßen und es kann nun die zweite Schraube eingesetzt werden. Die zweite Schraube wird also den Zapfen 27 querend eingesetzt oder aber seitlich neben diesem Zapfen, falls die Schrauben nicht in einer Ebene liegend oder sich unmittelbar kreuzend angeordnet sind. Der Zapfen 27 kann von vornherein eine in der Regel zu große Länge aufweisen und wird dann nach dem Setzen der zweiten Schraube abgelängt, d.h. es wird der frei vorstehende Bereich des Zapfens abgetrennt. Falls die zweite Schraube nur geringfügig oder praktisch gar nicht versenkt angeordnet ist, wird auf diese nur eine Art Abdeckkappe 26 aufgesetzt. Diese Abdeckkappe 26 kann auf verschiedenste Art und Weise konstruiert sein. So ist es denkbar, eine Ausführung ähnlich der Ausgestaltung nach Fig. 10 vorzusehen oder aber eine Ausführung nach Fig. 16 oder aber auch eine Ausführung mit gleichem Material wie beim Zapfen 27. Bei der Ausgestaltung nach Fig.16 ist ein in den Innenwerkzeugangriff 5 der Knochenschraube 1 eingreifender Bereich 35 vorgesehen, der beispielsweise zwei Abschnitte aufweist, welche nach dem Einsetzen elastisch federnd an der Innenwandung des Innenwerkzeugangriffes 5 anliegen. Diese Abdeckkappe 26 verschließt dadurch den Innenwerkzeugangriff 5 der Knochenschraube 1 und den eventuell noch freibleibenden Bereich der Bohrung für die Aufnahme der zweiten Knochenschraube.

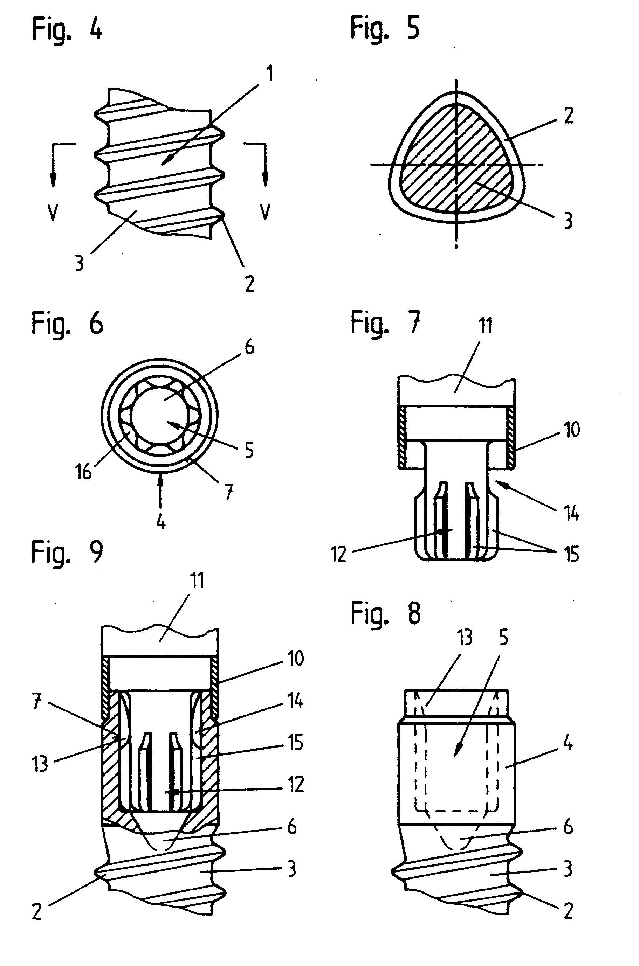

Wie aus den Fig. 7 bis 9 ersichtlich ist, wird der ringförmige Bund 7 an eine auf ein Werkzeug 11 aufgesetzte Ringmanschette 10 angepaßt, welche beim Schraubvorgang den ringförmigen Bund 7 des Kopfes 4 umfaßt. Die Knochenschraube 1 kann daher vor dem Einschraubvorgang an das Werkzeug 11 angesetzt werden, wo diese dann achsgleich ausgerichtet durch den Angriff 12 selbst und durch die koaxial dazu angeordnete Ringmanschette 10 gehalten wird. Die Angriffsrippen 15 des Angriffes 12 sind kürzer ausgeführt als die Tiefe des Innenwerkzeugangriffes 5 an der Knochenschraube 1, d.h. es wird hier eine umlaufende Nut 14 gebildet. Es ist daher eine besonders weiche Krafteinleitung möglich, auch wenn einmal der Angriff 12 nicht zur Gänze in den Innenwerkzeugangriff 5 eingeführt wäre. Die Angriffsrippen 16 in dem Innenwerkzeugangriff 5 sind zum freien Ende hin mit einer Abschrägung 13 versehen, um ein leichteres Einführen des Angriffes 12 ermöglichen zu können.

Die Fig. 10 bis 12 zeigen eine kraft- und/oder formschlüssig auf den Kopf 4 aufsetzbare Abdeckkappe 18 zum Verschließen des Innenwerkzeugangriffes 5. Die Abdeckkappe 18 weist einen in Richtung zum Kopf 4 gerichteten ringförmig verlaufenden Steg 19 auf, welcher auf den ringförmigen Bund 7 verringerten Durchmessers DB am Kopf 4 aufschiebbar ist. Der Außendurchmesser D der Abdeckkappe 18 ist zumindest annähernd gleich dem Außendurchmesser D1 des Kopfes 4. Damit die Abdeckkappe 18 mit einem Werkzeug 20 bzw. einem Fortsatz 21 eines solchen Werkzeuges 20 in einfacher Weise an der Knochenschraube 1 aufgesetzt werden kann, hat die Abdeckkappe 18 in Achsrichtung gesehen nach beiden Seiten hin abstehende ringförmig verlaufende Stege 19 und 22. Die Abdeckkappe 18 kann an ihrer Außenbegrenzung eine oder mehrere rillenartige Vertiefung(en) 23 aufweisen. Nach dem Aufsetzen der Abdeckkappe 18 auf den Kopf 4 der Knochenschraube 1 kann das schnell nachwachsende Knochengewebe auch in diese rillenartige(n) Vertiefung(en) 23 eindringen und so zusätzlich auch die Abdeckkappe 18 in ihrer Lage gegenüber dem Kopf 4 sichern.

Die Fig. 13 und 14 zeigen eine besondere Ausbildung der Abdeckkappe 18 und ein damit einsetzbares Werkzeug 24. Die Abdeckkappe 18 weist in ihrem Zentrum eine Solldurchbruchstelle und/oder z.B. sektorförmig über Sollbruchlinien verbundene Abschnitte zur Herstellung einer Öffnung beim Einsatz eines Werkzeuges 24 mit einer kegel- oder pyramidenförmigen Spitze 25 auf. Wenn also nach einiger Zeit die Knochenschraube 1 wieder entfernt, also herausgedreht werden soll, dann kann das Werkzeug 4 eingeführt werden, wobei die Spitze 25 am freien Ende dieses Werkzeuges 24 die Abdeckkappe 18 in ihrem Zentrum durchdringt. Die widerhakenartige Ausbildung des Werkzeuges 24 bewirkt, daß beim Zurückziehen desselben die Abdeckkappe 18 vom Kopf 4 der Knochenschraube 1 abgezogen wird. Nach dem Entfernen der Abdeckkappe 18 kann dann in einfacher Weise das Werkzeug 11 mit dem Angriff 12 und der Ringmanschette 10 angesetzt werden, da alle diese Bereiche für den Angriff des Werkzeuges 11 von der Abdeckkappe 18 abgeschlossen waren und nun wieder zugänglich sind. Ein Durchstoßen der Abdeckkappe ist auch direkt mittels eines Schraubendrehers möglich, so daß die Knochenschraube herausgedreht werden kann, ohne vorher die Abdeckkappe zu entfernen.

Bei der Ausgestaltung nach Fig. 15 geht es um die besondere Art eines Werkzeuges 11 zum Eindrehen der Knochenschraube 1. Der Durchmesser des Kopfes 4 der Schraube kann hier sogar noch etwas kleiner ausgeführt werden als der Außendurchmesser des Gewindes 2. Das in den Innenwerkzeugangriff 5 der Schraube einsetzbare Werkzeug 11 wird hier von einer Hülse 28 koaxial umschlossen, Diese Hülse 28 umgreift zumindest einen Großteil der axialen Länge des Kopfes 4 der Schraube koaxial und bietet daher für die Schraube einen festen Halt. Die Achsausrichtung zwischen Schraube und Werkzeug 11 ist dadurch optimal. Mittels einer Schulter 33 stützt sich die Hülse 28 am freien Ende des Kopfes 4 ab. Das rückwärtige Ende der Hülse findet durch einen vorspringenden Bund 29 od.dgl. am Werkzeug 11 einen Anschlag. Die Hülse 28 ist somit immer gleich bezüglich des Werkzeuges 11 ausgerichtet, so daß auch ein gleichbleibender Eingriff im Bereiche des Innenwerkzeugangriffes gewährleistet ist. Anstelle der Anordnung eines Bundes ist es auch denkbar, das Werkzeug 11 mit einem entsprechenden Anschlag zu versehen, also beispielsweise ab diesem Anschlagbereich im Durchmesser größer auszuführen. Es könnten dann beispielsweise die Hülse 28 und der auf die Hülse folgende Abschnitt des Werkzeuges den gleichen Außendurchmesser aufweisen. Der unmittelbar den Kopf 4 der Schraube umschließende Abschnitt 30 der Hülse 28 ist durch die besondere Konstruktion etwas dünner als der restliche Abschnitt der Hülse 28. Der Abschnitt 30 trägt dadurch nicht zuviel an Außendurchmesser auf, so daß die herzustellenden Bohrung nicht wesentlich größer sein muß als die für den Schaftbereich der Schraube. Für des bessere Einführen des freien Endes der Hülse 28 im Bereich der Bohrung ist es vorteilhaft, wenn das freie Ende des Abschnittes 30 der Hülse 28 mit einer Anfasung 31 versehen ist.

Um ein einfacheres Setzen ohne allzugroße Radialkräfte im Knochenbereich zu ermöglichen, kann gerade beim Einsatz eines Werkzeuges gemäß Fig. 15 eine andere Bohrtechnik eingesetzt werden. Es kann dann vorerst eine Bohrung in Anpassung an den Schaftbereich der Schraube hergestellt werden. Es ist dadurch auch eine exakte Wahl der erforderlichen Tiefe der Bohrung möglich. Zum optimalen Setzen der Schraube 1 insbesondere im Zusammenhang mit einem Werkzeug nach Fig. 15 ist jedoch eine Art Stufenbohrung sinnvoll. Es wird dann also als zweiter Bohrschritt eine Art Stufenbohrer eingesetzt. Auf einen auf den Bohrdurchmesser der Hauptbohrung angepaßten zylindrischen Bolzen folgt ein Bohrabschnitt, der im Durchmesser der Hülse 28 oder eben einem größeren Kopf der Schraube angepaßt ist.

Im Rahmen der Erfindung sind auch andere Gewinde/Schaft/Kombinationen möglich. Es können auch ganz spezielle Gewindeformen zum Einsatz kommen. Es wären auch zwei Gewindeabschnitte mit unterschiedlicher Gewindesteigung denkbar oder ein Gewindeabschnitt, in dessen Verlauf sich die Gewindesteigung ändert. Dies würde noch zusätzliche Varianten zur Verdrehsicherung und zur Längsarretierung mit sich bringen.

## Patentansprüche

1. Knochenschraube, insbesondere für den Einsatz bei translaminärer Wirbelverschraubung, mit einem Schaft sowie einem an wenigstens einem Ende ausgebildeten Kopf mit einem Werkzeugangriff, wobei der Schaft an dem dem Kopf zugewandten Endabschnitt einen mit einem Gewinde versehenen Bereich aufweist, **dadurch gekennzeichnet, dass** der auf den mit dem Gewinde (2) versehenen Bereich (A) folgende restliche Bereich (B) des Schaftes (3) bis zum freien Ende gewindefrei ausgeführt ist, dass der Kopf (4) einen zumindest annähernd dem Außendurchmesser (DA) des Gewindes (2) entsprechenden Durchmesser (D1) aufweist und mit einem Innenwerkzeugangriff (5) versehen ist, und dass der Bereich (A) des Schaftes (3) mit dem Gewinde (2) und/oder der gewindelose Bereich (B) des Schaftes (3) unrund, nach Art eines Gleichdicks, z.B. trilobular, oder mehreckig ausgestaltet und/oder mit Erhebungen und/oder Vertiefungen versehen ist.

2. Knochenschraube nach Anspruch 1, **dadurch gekennzeichnet, dass** das Gewinde (2) auf dem Schaft (3) selbstfurchend und/oder selbstschneidend ausgebildet ist.

3. Knochenschraube nach den Ansprüchen 1 und 2, **dadurch gekennzeichnet, dass** der gewindefreie Bereich (B) des Schaftes (3) zylindrisch oder konisch ausgeführt ist, wobei der Bereich (A) des Schaftes (3) mit dem Gewinde (2) unrund, nach Art eines Gleichdicks, z.B. trilobular, oder mehreckig ausgestaltet und/oder mit Erhebungen und/oder Vertiefungen versehen ist.

4. Knochenschraube nach den Ansprüchen 1 und 2, **dadurch gekennzeichnet, dass** das Gewinde (2) und der Schaft (3) in diesem Bereich (A) zylindrisch ausgeführt sind, wobei der an den Bereich (A) mit dem Gewinde (3) anschließende gewindefreie Bereich (B) des Schaftes (3) unrund, nach Art eines Gteichdicks, z.B. trilobular, oder mehreckig ausgestaltet und/oder mit Erhebungen und/oder Vertiefungen versehen ist.

5. Knochenschraube nach einem der Ansprüche 1 bis 4, **dadurch gekennzeichnet, dass** der Bereich (A) des Schaftes (3) mit dem Gewinde (2) unmittelbar an den Kopf (4) anschließend angeordnet ist.

6. Knochenschraube nach einem der Ansprüche 1 bis 5, **dadurch gekennzeichnet, dass** der Außendurchmesser (DG) des gewindefreien Bereiches (B) des Schaftes (3) zumindest annähernd dem Kerndurchmesser (DK) des Bereiches (A) mit dem Gewinde (2) entspricht.

7. Knochenschraube nach einem der Ansprüche 1 bis 6, **dadurch gekennzeichnet, dass** die axiale Länge des Bereiches (A) mit dem Gewinde (2) kleiner ist als die axiale Länge des gewindefreien Bereiches (B) des Schaftes (3).

8. Knochenschraube nach einem der Ansprüche 1 bis 7, **dadurch gekennzeichnet, dass** der Innenwerkzeugangriff (5) am Kopf (4) an dessen tiefster Begrenzung eine zusätzliche Ausnehmung oder eine taschenartige Vertiefung (6) aufweist.

9. Knochenschraube nach einem der Ansprüche 1 bis 8, **dadurch gekennzeichnet, dass** der Kopf (4) an seinem freien Ende einen ringförmigen Bund (7) mit gegenüber dem Außendurchmesser (D1) des Kopfes (4) verringertem Durchmesser (DB) aufweist.

10. Knochenschraube nach Anspruch 9, **dadurch gekennzeichnet, dass** die axiale Länge (L) des ringförmigen Bundes (7) weniger als die halbe Gesamtlänge (K), vorzugsweise weniger als ein Drittel der Gesamtlänge (K) des Kopfes (4) beträgt.

11. Knochenschraube nach den Ansprüchen 9 und 10, **dadurch gekennzeichnet, dass** der ringförmige Bund (7) an eine auf ein Werkzeug (20) aufgesetzte Ringmanschette (10) angepasst ist, welche beim Schraubvorgang den ringförmigen Bund (7) des Kopfes (4) umfasst.

12. Knochenschraube nach einem der Ansprüche 1 bis 11, **dadurch gekennzeichnet, dass** am freien Ende des gewindefreien Bereiches (B) des Schaftes (3) ein z. B. kegelstumpf- oder halbkugelförmig sich verjüngender Abschnitt (17) ausgebildet ist.

13. Knochenschraube nach einem der Ansprüche 1 bis 12, **gekennzeichnet durch** eine kraftund/oder formschlüssig auf den Kopf (4) aufsetzbare Abdeckkappe (18) zum Verschließen des Innenwerkzeugangriffes (5).

14. Knochenschraube nach den Ansprüchen 9 und 13, **dadurch gekennzeichnet, dass** die Abdeckkappe (18) einen in Richtung zum Kopf (4) weisenden ringförmig verlaufenden Steg (19) aufweist, welcher auf den ringförmigen Bund (7) verringerten Durchmessers (DB) am Kopf (4) aufschiebbar ist.

15. Knochenschraube nach den Ansprüchen 13 und 14, **dadurch gekennzeichnet, dass** der Außendurchmesser (D) der Abdeckkappe (18) zumindest annähernd gleich ist dem Außendurchmesser (D1) des Kopfes (4).

16. Knochenschraube nach den Ansprüchen 13 bis 15, **dadurch gekennzeichnet, dass** die Abdeckkappe (18) in Achsrichtung gesehen nach beiden Seiten hin abstehende ringförmig verlaufende Stege (19,22) aufweist.

17. Knochenschraube nach den Ansprüchen 13 bis 16, **dadurch gekennzeichnet, dass** die Abdeckkappe (18) in ihrem Zentrum eine Solldurchbruchstelle und/oder z.B. sektorförmig über Sollbruchlinien verbundene Abschnitte zur Herstellung einer Öffnung beim Einsatz eines Werkzeuges (24) mit einer kegel- oder pyramidenförmigen Spitze (25) aufweist.

18. Knochenschraube nach den Ansprüchen 13 bis 17, **dadurch gekennzeichnet, dass** die Abdeckkappe (18) an ihrer Außenbegrenzung einen oder mehrere rillenartige Vertiefungen (23) aufweist.

## Claims

1. A bone screw, in particular for use in a translaminar vertebral screw connection, having a shank with a head formed at at least one end and with a tool engagement portion, wherein at the end portion nearest the head the shank has a zone provided with a screw-thread, **characterised in that** the remaining zone (B) of the shank (3) following the zone (A) provided with the screw-thread (2) is formed threadless up to the free end, **in that** the head (4) is of a diameter (D1) corresponding at least approximately to the outer diameter (DA) of the screw-thread (2) and is provided with an internal tool engagement portion (5), and **in that** the zone (A) of the shank (3) with the screw-thread (2) and/or the threadless zone (B) of the shank (3) is of non-circular, constant-diameter type, for example trilobular, or of polygonal conformation and/or is provided with projections and/or indentations.

2. A bone screw according to Claim 1, **characterised in that** the screw-thread (2) on the shank (3) is designed to be thread-forming and/or thread-cutting.

3. A bone screw according to Claims 1 and 2, **characterised in that** the threadless zone (B) of the shank (3) is of cylindrical or conical form, wherein the zone (A) of the shank (3) with the screw-thread (2) is of non-circular, constant-diameter type, for example trilobular, or of polygonal conformation and/or is provided with projections and/or indentations.

4. A bone screw according to Claims 1 and 2, **characterised in that** the screw-thread (2) and the shank (3) in this zone (A) are of cylindrical form, wherein the threadless zone (B) of the shank (3) adjacent the zone (A) with the screw-thread (3) is of non-circular, constant-diameter type, for example trilobular, or of polygonal conformation and/or is provided with projections and/or indentations.

5. A bone screw according to any one of Claims 1 to 4, **characterised in that** the zone (A) of the shank (3) with the screw-thread (2) is arranged immediately adjacent the head (4) .

6. A bone screw according to any one of Claims 1 to 5, **characterised in that** the outer diameter (DG) of the threadless zone (B) of the shank (3) corresponds at least approximately to the root diameter (DK) of the zone (A) with the screw-thread (2).

7. A bone screw according to any one of Claims 1 to 6, **characterised in that** the axial length of the zone (A) with the screw-thread (2) is smaller than the axial length of the threadless zone (B) of the shank (3).

8. A bone screw according to any one of Claims 1 to 7, **characterised in that** the internal tool engagement portion (5) on the head (4) has at its deepest boundary an additional recess or a pocket-like indentation (6).

9. A bone screw according to any one of Claims 1 to 8, **characterised in that** at its free end the head (4) has an annular flange (7) of reduced diameter (DB) in relation to the outer diameter (D1) of the head (4).

10. A bone screw according to Claim 9, **characterised in that** the axial length (L) of the annular flange (7) is less than half the total length (K), preferably less than one third of the total length (K) of the head (4).

11. A bone screw according to Claims 9 and 10, **characterised in that** the annular flange (7) is adapted to a annular collar (10) which is fitted on a tool (20) and which during the screwing operation encloses the annular flange (7) of the head (4).

12. A bone screw according to any one of Claims 1 to 11, **characterised in that** at the free end of the threadless zone (B) of the shank (3) there is formed, for example, a frustoconically or hemispherically tapering portion (17).

13. A bone screw according to any one of Claims 1 to 12, **characterised by** a covering cap (18), which can fitted in force-locking or force-locking manner on the head (4), for closing the internal tool engagement portion (5).

14. A bone screw according to Claims 9 and 13, **characterised in that** the covering cap (18) has an annularly extending web (19) which faces towards the head (4) and which can be fitted on to the annular flange (7) of reduced diameter (DB) on the head (4).

15. A bone screw according to Claims 13 and 14, **characterised in that** the outer diameter (D) of the covering cap (18) is at least approximately equal to the outer diameter (D1) of the head (4).

16. A bone screw according to any one of Claims 13 to 15, **characterised in that**, viewed in an axial direction, the covering cap (18) has annularly extending webs (19,22) protruding on both sides.

17. A bone screw according to any one of Claims 13 to 16, **characterised in that** in its centre the covering cap (18) has a predetermined breaking point and/or portions joined, for example in sector-like manner, via predetermined breaking lines so as to establish an opening when using a tool (24) having a conical or pyramid-shaped tip (25).

18. A bone screw according to any one of Claims 13 to 17, **characterised in that** at its outer boundary the covering cap (18) has one or more groove-like indentations (23).

## Revendications

1. Vis pour fracture osseuse, en particulier pour utilisation dans le vissage translaminé entre une vertèbre, comportant un arbre ainsi qu'une tête formée à au moins une extrémité avec une prise d'instrument, où l'arbre présente sur la section d'extrémité dirigée vers la terre une zone munie d'un filetage,
**caractérisée en ce que**
la zone (B) de l'arbre (3) subsistant à la suite de la zone (A) munie du filetage (2) est réalisée sans filetage jusqu'à l'extrémité libre, la tête (4) présente un diamètre (DA) correspondant au moins approximativement au diamètre externe (DA) du filetage (2) et est muni d'une prise d'instrument interne (5), et la zone (A) de l'arbre (3) avec le filetage (2) et/ou la zone sans filetage (B) de l'arbre (3) est de forme non arrondie, du type d'un cylindre, par exemple trilobulaire, ou polygonal et/ou est munie de saillies et/ou d'évidements.

2. Vis pour fracture osseuse selon la revendication 1,
**caractérisée en ce que**
le filetage (2) sur l'arbre (3) est formé de manière à créer par lui-même des sillons et/ou est autocoupant.

3. Vis pour fracture osseuse selon les revendications 1 et 2,
**caractérisée en ce que**
la zone sans filetage (B) de l'arbre (3) est de forme cylindrique ou conique, la zone (A) de l'arbre (3) avec le filetage (2) étant de forme non arrondie, du type d'un cylindre, par exemple trilobulaire ou polygonal et/ou étant munie de saillies et/ou d'évidements.

4. Vis pour fracture osseuse selon les revendications 1 et 2,
**caractérisée en ce que**
le filetage (2) et l'arbre (3) dans cette zone (A) sont de forme cylindrique, la zone (B) sans filetage de l'arbre (3) faisant suite à la zone (A) avec le filetage (3) étant non arrondie, du type d'un cylindre, par exemple trilobulaire, ou de forme polygonale, et/ou étant munie de saillies et/ou d'évidements.

5. Vis pour fracture osseuse selon l'une des revendications 1 à 4,
**caractérisée en ce que**
la zone (A) de l'arbre (3) avec le filetage (2) est disposée immédiatement à la suite de la tête (4).

6. Vis pour fracture osseuse selon l'une des revendications 1 à 5,
**caractérisée en ce que**
le diamètre externe (DG) de la zone sans filetage (B) de l'arbre (3) correspond de manière au moins approximative au diamètre du noyau (DK) de la zone (A) avec le filetage (2).

7. Vis pour fracture osseuse selon l'une des revendications 1 à 6,
**caractérisée en ce que**
la longueur axiale de la zone (A) avec le filetage (2) est inférieure à la longueur axiale de la zone sans filetage (B) de l'arbre (3).

8. Vis pour fracture osseuse selon l'une des revendications 1 à 7,
**caractérisée en ce que**
l'attaque pour instruments internes (5) sur la tête (4) présente à sa limite la plus profonde un évidement supplémentaire ou un trou en forme de poche (6).

9. Vis pour fracture osseuse selon l'une des revendications 1 à 8,
**caractérisée en ce que**
la tête (4) présente à son extrémité libre une collerette annulaire (7) avec diamètre réduit (DB) par rapport au diamètre externe (D1) de la tête (4).

10. Vis pour fracture osseuse selon la revendication 9,
**caractérisée en ce que**
la longueur axiale (L) de la collerette annulaire (7) est inférieure à la moitié de la longueur totale (K), de préférence inférieure à 1/3 de la longueur totale K de la tête (4).

11. Vis pour fracture osseuse selon les revendicatioins 9 et 10,
**caractérisée en ce que**
la collerette annulaire (7) est adaptée à une manchette annulaire (10) disposée sur un instrument (20), qui comprend lors du processus de vissage la collerette annulaire (7) de la tête (4).

12. Vis pour fracture osseuse selon l'une des revendications 1 à 11,
**caractérisée en ce qu'**
à l'extrémité libre de la zone sans filetage de l'arbre (3) est formé un segment (17) en forme de cylindre tronqué ou de semi-cylindre qui se rétrécit.

13. Vis pour fracture osseuse selon l'une des revendications 1 à 12,
**caractérisée par**
un capuchon (18) pouvant être fixé au moyen d'une liaison par la force et/ou par la forme sur la tête (4) afin de sceller la prise d'instrument interne (5).

14. Vis de fracture osseuse selon les revendications 9 et 13,
**caractérisée en ce que**
le capuchon (18) présente une nervure (19) courant de manière annulaire, qui peut être encagée sur la collerette annulaire (7) de diamètre diminué (DB) sur la tête (4).

15. Vis de fracture osseuse selon les revendications 13 et 14,
**caractérisée en ce que**
le diamètre externe (D) du capuchon (18) est au moins approximativement identique au diamètre externe (D1) de la tête (4).

16. Vis pour fracture osseuse selon la revendications 13 à 15,
**caractérisée en ce que**
le couvercle de revêtement (18) présente dans la direction de l'axe des nervures courant de manière annulaire sur les deux côtés à un certain intervalle.

17. Vis pour fracture osseuse selon les revendications 13 à 16,
**caractérisée en ce que**
le capuchon (18) présente en son centre un point destiné à la rupture et/ou des segments en forme de secteurs reliés par des lignes de rupture en vue de produire une ouverture par introduction d'un instrument (24) ayant une pointe conique ou pyramidale (25).

18. Vis pour fracture osseuse selon les revendications 13 à 17,
**caractérisée en ce que**
le capuchon (18) présente à sa limite externe un ou plusieurs creux (23) en forme de rainures.
